# EUROPEAN PATENT APPLICATION

(11) **EP 4 810 019 A1**
(43) Date of publication of application: **23.09.2026**
(21) Application number: 26165940.3
(22) Date of filing: 19.03.2026
(51) Int. Cl.: A61K 9/107, A61K 47/10, A61K 47/12, A61K 47/26, A61K 31/122

(54) **PHYTONADIONE EMULSION**

(30) Priority: 19.03.2025 US 202519083869
(71) Applicant: B. Braun Melsungen AG, 34212 Melsungen (DE)
(72) Inventor: ETHIER, Anabelle, Sainte-Victoire-de-Sorel, J0G1T0 (CA); GENDRON, Marie-Claude, Saint-Hyacinthe, J2R1C7 (CA); LAJOIE, Valérie, Saint-Hyacinthe, J2T5E2 (CA); HANDFIELD, Maxim, Blainville, J7C 0L3 (CA)
(74) Representative: Maiwald GmbH

(57) **Abstract**

A pharmaceutical emulsion composition contains phytonadione, a non-ionic surfactant, and an emulsifier in water. The pharmaceutical emulsion can be manufactured by a method that includes the step of mixing phytonadione and a non-ionic surfactant. The method can be used to prepare a pharmaceutical composition, which can be packaged in a container, such as a syringe.

## Description

### FIELD

The present disclosure relates to a pharmaceutical emulsion composition comprising phytonadione, a non-ionic surfactant, and an emulsifier in water, and a method of manufacturing such pharmaceutical emulsion composition, as well as a pharmaceutical composition prepared by said method and a container comprising the pharmaceutical composition according to the present disclosure.

### BACKGROUND

Phytonadione, also known as vitamin K1, is a fat-soluble vitamin that plays a critical role in various physiological processes. Its primary uses include the treatment and/or prevention of vitamin K deficiency, which can result in impaired blood clotting and bleeding disorders.

Furthermore, phytonadione may be administered to counteract excessive anticoagulation caused by an overdose of warfarin or other vitamin K antagonist drugs. This is critical in preventing or controlling bleeding episodes.

Phytonadione may also be routinely given to newborns as a single intramuscular injection to prevent hemorrhagic disease of the newborn (HDN), a condition caused by low levels of vitamin K at birth.

Further uses include conditions such as hypoprothrombinemia (low levels of prothrombin in the blood) due to vitamin K deficiency, and/or liver disease-related coagulopathy, where clotting factors are deficient, and/or alongside other treatments in conditions involving malabsorption syndromes (e.g., celiac disease, Crohn's disease) where the body cannot properly absorb fat-soluble vitamins, including vitamin K.

Routes of administration usually involve oral administration for mild cases of deficiency or preventive measures, intravenous (IV) administration for urgent reversal of bleeding or severe deficiency, and subcutaneous or intramuscular administration for prophylaxis or in patients unable to take oral medications.

In particular, for intravenous administration, prefilled syringes are preferred to administer phytonadione emulsions since they do not require any type of dilution or breaking a glass ampule for application. During emergency conditions, prefilled syringes are very useful and quick action to the patients. This increases medication safety, as the dosage is more stable and precise, and sterility is maintained as less manipulation is needed from users. The required preparation of the medication is also lowered for nurses (or any other authorized user) in the hospital because the syringe is already filled and ready to administer.

On the other hand, storing ready-to-administer phytonadione (vitamin K1) compositions involves specific challenges due to the compound's sensitivity to environmental factors and its chemical properties.

Phytonadione is highly sensitive to light (photosensitive), leading to degradation and reduced potency when exposed to light. Therefore, storage in amber-colored vials is needed which are difficult to inspect visually.

Furthermore, exposure to oxygen can cause oxidation of phytonadione, decreasing its effectiveness. Thus, packaging the product in airtight containers and minimizing exposure to air during preparation and administration is required.

When prepared in an aqueous solution, phytonadione is described to be particularly prone to instability, which can lead to precipitation or chemical breakdown over time.

Also, phytonadione can adsorb to the surface of glass or plastic containers, especially in aqueous solutions, leading to a reduction in the available dose.

Often, phytonadione is formulated as an oil-in-water emulsion to improve its poor water solubility. Over time, emulsions tend to separate forming a two-phase system, affecting dose uniformity and efficacy.

In conclusion, formulations of phytonadione, in particular ready-to-administer formulations of phytonadione, are particularly difficult to store. Degradation over time can reduce the potency of phytonadione, particularly in prefilled syringes or diluted solutions.

To ensure the efficacy and safety of ready-to-administer phytonadione compositions, it is essential to store them in light-resistant containers, at appropriate temperatures, with attention to preventing oxidation, microbial contamination, and emulsion instability. Following the manufacturer's guidelines and using the product promptly after preparation are critical.

### SUMMARY

Therefore, there is an urgent need for phytonadione compositions that overcome the above-mentioned drawbacks of known formulations.

The present disclosure at least partially solves the above problems.

According to a first aspect, the present disclosure relates to a pharmaceutical composition comprising a) phytonadione, b) a non-ionic surfactant, c) an emulsifier; and d) water for injection, wherein the ratio: $\frac{weight \left(non-ionic surfactant\right)}{weight \left(phytonadione\right)}$ is in the range of from about 5 to about 20, and wherein the pharmaceutical composition comprises a plurality of association colloids having a mean particle size in the range of from about 10 nm to about 20 nm.

**In** a second aspect, the present disclosure is directed to a method of manufacturing the pharmaceutical composition according to the first aspect of the present disclosure comprising the steps of
a) mixing phytonadione and a non-ionic surfactant to provide a first mixture, and heating said first mixture to a first temperature T₁ to provide a first mixture having a temperature T₁, wherein T₁ is in the range of from about 50 °C to about 80 C;
b) dissolving an emulsifier in water for injection to provide a second mixture, and heating said second mixture to a second temperature T₂ to provide a second mixture having a temperature T₂, wherein T₂ is in the range of from about 50 °C to about 80 °C;
c) mixing said first mixture having a temperature T₁ obtained from step a) and said second mixture having a temperature T₂ obtained from step b) to provide a third mixture; and
d) cooling down said third mixture to a temperature T₃ in the range of from about 20 °C to about 30 °C to provide the pharmaceutical composition according to the first aspect of the present disclosure.

In a third aspect, the present disclosure relates to a pharmaceutical composition obtained by the method according to the second aspect of the present disclosure.

According to a fourth aspect, the present disclosure relates to a container comprising the pharmaceutical composition according to the first aspect of the present disclosure or the pharmaceutical composition according to the third aspect of the present disclosure.

### BRIEF DESCRIPTION OF THE FIGURES

**Fig. 1****:** Results of stability studies (assay of phytonadione in composition No. 5 of example 1) upon storage at 25±2 °C / 60±5 % RH, 30±2 °C / 65±5 % RH, and 40±2 °C / 75±5 % RH, respectively, as determines in example 5 of the present disclosure.
**Fig. 2****:** Results of stability studies (assay of total impurities in composition No. 5 of example 1) upon storage at 25±2 °C / 60±5 % RH, 30±2 °C / 65±5 % RH, and 40±2 °C / 75±5 % RH, respectively, as determines in example 5 of the present disclosure.
**Fig. 3****:** Results of photo-stability and oxygen stability studies of example 3 (assay of hydroperoxy-phytonadione in composition No. 5 of example 1). The graph shows the level of hydroperoxy-phytonadione determined according to method (3) as discloses herein minus the level of hydroperoxy-phytonadione determined according to method (3) at 0 mins.

### DETAILED DESCRIPTION

In the following, the present disclosure will be further explained with reference to preferred embodiments.

### The pharmaceutical composition

According to a first aspect, the present disclosure relates to a pharmaceutical composition comprising a) phytonadione, b) a non-ionic surfactant, c) an emulsifier; and d) water for injection, wherein the ratio: $\frac{weight \left(non-ionic surfactant\right)}{weight \left(phytonadione\right)}$ is in the range of from about 5 to about 20, and wherein the pharmaceutical composition comprises a plurality of association colloids having a mean particle size in the range of from about 10 nm to about 20 nm.

The inventors of the present disclosure have surprisingly found how to prepare a pharmaceutical composition comprising a plurality of association colloids having a mean particle size in the range of from about 10 nm to about 20 nm. As is shown in example 1, the pharmaceutical composition according to the first aspect of the present disclosure may be advantageously prepared by a phase inversion emulsification process.

Without wishing to be bound by theory, the association colloids are formed by the non-ionic surfactant, such as Polysorbate 80 (PS80), and phytonadione is stabilized inside the association colloids. In the pharmaceutical composition, the emulsifier may also associate with the association colloid and further stabilize the association colloids from aggregation and decomposition.

When administered to a subject, such as a human or mammal, phytonadione is released from the association colloids and act therapeutically.

It has been surprisingly found that the pharmaceutical composition comprising a plurality of association colloids is highly stable, i.e., has an extended shelf life (cf. example 5), and phytonadione is stabilized against external influences, such as against light and oxygen stress (see example 3).

Also, the pharmaceutical compositions according to the first aspect of the present disclosure also has a viscosity that is close to the viscosity of water. Therefore, the pharmaceutical compositions can be readily administered intravenously or by other injection methods as shown in example 4. Therefore, the pharmaceutical composition according to the first aspect can be advantageously used for injections and can be advantageously stored in prefilled syringes as disclosed herein.

According to a preferred embodiment of the first aspect of the present disclosure, the pharmaceutical composition has a viscosity of about 2.0 cP or less. According to another preferred embodiment of the first aspect of the present disclosure, the pharmaceutical composition has a viscosity of about 1.7 cP or less. According to another preferred embodiment of the first aspect of the present disclosure, the pharmaceutical composition has a viscosity of about 1.5 cP or less. According to another preferred embodiment of the first aspect of the present disclosure, the pharmaceutical composition has a viscosity of about 1.3 cP or less. According to a further preferred embodiment of the first aspect of the present disclosure, the pharmaceutical composition has a viscosity of about 1.2 cP or less.

According to a preferred embodiment of the first aspect of the present disclosure, the pharmaceutical composition has a viscosity in the range of from about 0.8 cp to about 2.0 cP. According to another preferred embodiment of the first aspect of the present disclosure, the pharmaceutical composition has a viscosity in the range of from about 0.9 cp to about 1.7 cP. According to another preferred embodiment of the first aspect of the present disclosure, the pharmaceutical composition has a viscosity in the range of from about 0.9 cP to about 1.5 cP. According to another preferred embodiment of the first aspect of the present disclosure, the pharmaceutical composition has a viscosity in the range of from about 0.9 cP to about 1.3 cP. According to a further preferred embodiment of the first aspect of the present disclosure, the pharmaceutical composition has a viscosity in the range of from about 1.0 cP to about 1.2 cP.

The inventors of the present disclosure have surprisingly found that the viscosity of the pharmaceutical composition according to the first aspect of the present disclosure has a viscosity that is close to water.

This is highly surprising because the presence of association colloids is expected to increase the viscosity of a composition as association colloids interact with each other or form an entangled network structure.

According to a preferred embodiment of the present disclosure, the pharmaceutical composition comprises a plurality of association colloids having a mean particle size is in the range of from about 10 nm to about 15 nm. According to another preferred embodiment of the present disclosure, the pharmaceutical composition comprises a plurality of association colloids having a mean particle size is in the range of from about 10 nm to about 13 nm. According to a further preferred embodiment of the present disclosure, the pharmaceutical composition comprises a plurality of association colloids having a mean particle size is about 10 nm or about 13 nm.

The inventors of the present disclosure have surprisingly found that pharmaceutical compositions comprising a plurality of association colloids with a particularly low viscosity can be prepared. Thus, small association colloids, such as micelles, efficiently solubilize poorly water-soluble drugs, improving their bioavailability. Their high surface-area-to-volume ratio allows for effective encapsulation of hydrophobic compounds.

Also, small association colloids are less likely to be recognized by the immune system, reducing the potential for immunogenic responses compared to larger delivery systems. Furthermore, smaller association colloids are more dynamic and can rapidly equilibrate between monomers and aggregates, which may facilitate controlled release of the drug.

However, the inventors of the present disclosure have surprisingly found that the pharmaceutical composition is highly stable upon storage and protects phytonadione from precipitation, from oxidation, and from light as shown in examples 3, 5, and 6.

According to a preferred embodiment of the present disclosure, the non-ionic surfactant is a polysorbate. According to a further preferred embodiment of the present disclosure, the non-ionic surfactant is polysorbate 80 (PS80).

According to another preferred embodiment of the present disclosure, the pharmaceutical composition according to the first aspect comprises phytonadione in a concentration of about 2 mg/ml. This concentration of phytonadione makes the pharmaceutical composition suitable for intravenous injection without the need for further processing. In other words, the pharmaceutical composition according to the first aspect of the present disclosure is a ready-to-administer formulation.

According to a preferred embodiment of the present disclosure, the pharmaceutical composition comprises the non-ionic surfactant in a concentration in the range of from about 5 mg/ml to about 50 mg/ml. According to another preferred embodiment of the present disclosure, the pharmaceutical composition comprises the non-ionic surfactant in a concentration in the range of from about 10 mg/ml to about 40 mg/ml. According to another preferred embodiment of the present disclosure, the pharmaceutical composition comprises the non-ionic surfactant in a concentration in the range of from about 15 mg/ml to about 30 mg/ml. According to a further preferred embodiment of the present disclosure, the pharmaceutical composition comprises the non-ionic surfactant in a concentration in the range of from about 15 mg/ml to about 25 mg/ml.

According to another preferred embodiment of the present disclosure, the pharmaceutical composition comprises the non-ionic surfactant in a concentration of about 5 mg/ml or more. According to another preferred embodiment of the present disclosure, the pharmaceutical composition comprises the non-ionic surfactant in a concentration of about 10 mg/ml or more. According to another preferred embodiment of the present disclosure, the pharmaceutical composition comprises the non-ionic surfactant in a concentration of about 15 mg/ml or more. According to a further preferred embodiment of the present disclosure, the pharmaceutical composition comprises the non-ionic surfactant in a concentration of about 20 mg/ml.

According to another preferred embodiment of the present disclosure, the pharmaceutical composition comprises the non-ionic surfactant in a concentration of about 50 mg/ml or less. According to another preferred embodiment of the present disclosure, the pharmaceutical composition comprises the non-ionic surfactant in a concentration of about 40 mg/ml or less. According to another preferred embodiment of the present disclosure, the pharmaceutical composition comprises the non-ionic surfactant in a concentration of about 30 mg/ml or less. According to a further preferred embodiment of the present disclosure, the pharmaceutical composition comprises the non-ionic surfactant in a concentration of about 25 mg/ml or less.

According to a preferred embodiment of the present disclosure, the pharmaceutical composition of the first aspect comprises a non-ionic surfactant and phytonadione in a ratio $\frac{weight \left(non-ionic surfactant\right)}{weight \left(phytonadione\right)}$ in the range of from about 6 to about 15. According to a preferred embodiment of the present disclosure, the pharmaceutical composition of the first aspect comprises a non-ionic surfactant and phytonadione in a ratio $\frac{weight \left(non-ionic surfactant\right)}{weight \left(phytonadione\right)}$ in the range of from about 8 to about 15.

In other words, the pharmaceutical composition according to the first aspect of the present times comprises the non-ionic surfactant in an amount (in mg) that is at least 6 times or at least 8 times and no more than 15 times the amount (in mg) of phytonadione. The pharmaceutical composition comprises more non-ionic surfactant than phytonadione.

According to a further preferred embodiment of the present disclosure, the pharmaceutical composition according to the present disclosure comprises the non-ionic surfactant and phytonadione in a ratio $\frac{weight \left(non-ionic surfactant\right)}{weight \left(phytonadione\right)}$ of about 10.

According to a preferred embodiment of the present disclosure, the pharmaceutical composition of the first aspect comprises a non-ionic surfactant and phytonadione in a ratio $\frac{weight \left(non-ionic surfactant\right)}{weight \left(phytonadione\right)}$ of about 5 or more. According to another preferred embodiment of the present disclosure, the pharmaceutical composition of the first aspect comprises a nonionic surfactant and phytonadione in a ratio $\frac{weight \left(non-ionic surfactant\right)}{weight \left(phytonadione\right)}$ of about 6 or more. According to a further preferred embodiment of the present disclosure, the pharmaceutical composition of the first aspect comprises a non-ionic surfactant and phytonadione in a ratio $\frac{weight \left(non-ionic surfactant\right)}{weight \left(phytonadione\right)}$ of about 8 or more.

According to a preferred embodiment of the present disclosure, the pharmaceutical composition of the first aspect comprises a non-ionic surfactant and phytonadione in a ratio $\frac{weight \left(non-ionic surfactant\right)}{weight \left(phytonadione\right)}$ of about 20 or less. According to a preferred embodiment of the present disclosure, the pharmaceutical composition of the first aspect comprises a non-ionic surfactant and phytonadione in a ratio $\frac{weight \left(non-ionic surfactant\right)}{weight \left(phytonadione\right)}$ of about 15 or less. According to a preferred embodiment of the present disclosure, the pharmaceutical composition of the first aspect comprises a non-ionic surfactant and phytonadione in a ratio $\frac{weight \left(non-ionic surfactant\right)}{weight \left(phytonadione\right)}$ of about 12 or less.

According to another preferred embodiment of the present disclosure, the emulsifier is C₂-C₄ diol. According to another preferred embodiment of the present disclosure the emulsifier is ethylene glycol or propylene glycol (propane-1,2-diol). According to a further preferred embodiment of the present disclosure, the emulsifier is propylene glycol (propane-1,2-diol).

According to a preferred embodiment of the present disclosure, the pharmaceutical composition comprises the emulsifier in a concentration in the range of from about 5 mg/ml to about 50 mg/ml. According to another preferred embodiment of the present disclosure, the pharmaceutical composition comprises the emulsifier in a concentration in the range of from about 10 mg/ml to about 40 mg/ml. According to another preferred embodiment of the present disclosure, the pharmaceutical composition comprises the emulsifier in a concentration in the range of from about 15 mg/ml to about 30 mg/ml. According to a further preferred embodiment of the present disclosure, the pharmaceutical composition comprises the emulsifier in a concentration in the range of from about 15 mg/ml to about 25 mg/ml.

According to another preferred embodiment of the present disclosure, the pharmaceutical composition comprises the emulsifier in a concentration of about 5 mg/ml or more. According to another preferred embodiment of the present disclosure, the pharmaceutical composition comprises the emulsifier in a concentration of about 10 mg/ml or more. According to another preferred embodiment of the present disclosure, the pharmaceutical composition comprises the emulsifier in a concentration of about 15 mg/ml or more. According to a further preferred embodiment of the present disclosure, the pharmaceutical composition comprises the emulsifier in a concentration of about 20 mg/ml.

According to another preferred embodiment of the present disclosure, the pharmaceutical composition comprises the emulsifier in a concentration of about 50 mg/ml or less. According to another preferred embodiment of the present disclosure, the pharmaceutical composition comprises the emulsifier in a concentration of about 40 mg/ml or less. According to another preferred embodiment of the present disclosure, the pharmaceutical composition comprises the emulsifier in a concentration of about 30 mg/ml or less. According to a further preferred embodiment of the present disclosure, the pharmaceutical composition comprises the emulsifier in a concentration of about 25 mg/ml or less.

According to a preferred embodiment of the present disclosure, the pharmaceutical composition of the first aspect comprises a non-ionic surfactant and an emulsifier in a ratio $\frac{weight \left(non-ionic surfactant\right)}{weight \left(emulsifier\right)}$ of about 0.5 or more. According to another preferred embodiment of the present disclosure, the pharmaceutical composition of the first aspect comprises a non-ionic surfactant and an emulsifier in a ratio $\frac{weight \left(non-ionic surfactant\right)}{weight \left(emulsifier\right)}$ of about 0.7 or more. According to a further preferred embodiment of the present disclosure, the pharmaceutical composition of the first aspect comprises a non-ionic surfactant and an emulsifier in a ratio $\frac{weight \left(non-ionic surfactant\right)}{weight \left(emulsifier\right)}$ of about 0.8 or more.

According to a preferred embodiment of the present disclosure, the pharmaceutical composition of the first aspect comprises a non-ionic surfactant and an emulsifier in a ratio $\frac{weight \left(non-ionic surfactant\right)}{weight \left(emulsifier\right)}$ of about 2.0 or less. According to another preferred embodiment of the present disclosure, the pharmaceutical composition of the first aspect comprises a nonionic surfactant and an emulsifier in a ratio $\frac{weight \left(non-ionic surfactant\right)}{weight \left(emulsifier\right)}$ of about 1.5 or less. According to a further preferred embodiment of the present disclosure, the pharmaceutical composition of the first aspect comprises a non-ionic surfactant and an emulsifier in a ratio $\frac{weight \left(non-ionic surfactant\right)}{weight \left(emulsifier\right)}$ of about 1.2 or less.

According to a preferred embodiment of the present disclosure, the pharmaceutical composition of the first aspect comprises a non-ionic surfactant and an emulsifier in a ratio $\frac{weight \left(non-ionic surfactant\right)}{weight \left(emulsifier\right)}$ in the range of from about 0.5 to about 2.0. According to another preferred embodiment of the present disclosure, the pharmaceutical composition of the first aspect comprises a non-ionic surfactant and an emulsifier in a ratio $\frac{weight \left(non-ionic surfactant\right)}{weight \left(emulsifier\right)}$ in the range of from about 0.7 to about 1.5. According to a further preferred embodiment of the present disclosure, the pharmaceutical composition of the first aspect comprises a nonionic surfactant and an emulsifier in a ratio $\frac{weight \left(non-ionic surfactant\right)}{weight \left(emulsifier\right)}$ in the range of from about 0.8 to about 1.2. According to a yet further preferred embodiment of the present disclosure, the pharmaceutical composition of the first aspect comprises a non-ionic surfactant and an emulsifier in a ratio $\frac{weight \left(non-ionic surfactant\right)}{weight \left(emulsifier\right)}$ of about 1.0.

According to another preferred embodiment of the present disclosure, the pharmaceutical composition according to the first aspect has a phase inversion temperature in the range of from about 50 °C to about 80 °C.

According to another preferred embodiment of the present disclosure, the pharmaceutical composition according to the first aspect has a phase inversion temperature in the range of from about 60 °C to about 70 °C.

According to another preferred embodiment of the present disclosure, the pharmaceutical composition according to the first aspect has a phase inversion temperature in the range of from about 60 °C to about 65 °C.

According to another preferred embodiment of the present disclosure, the pharmaceutical composition comprises a plurality of association colloids having a SPAN of in the range of from about 0.6 to about 0.9.

According to another preferred embodiment of the present disclosure, the pharmaceutical composition comprises a plurality of association colloids having a SPAN in the range of from about 0.65 to about 0.8.

According to another preferred embodiment of the present disclosure, the pharmaceutical composition comprises a plurality of association colloids having a SPAN in the range of from about 0.65 to about 0.75.

According to a further preferred embodiment of the present disclosure, the pharmaceutical composition comprises a plurality of association colloids having a SPAN of about 0.7.

According to another preferred embodiment of the present disclosure, the pharmaceutical composition according to the first aspect is in the form of a microemulsion.

According to another preferred embodiment of the present disclosure, the pharmaceutical composition according to the first aspect comprises a plurality of association colloids having a polydispersity index (PI) of less than 0.1.

In other words, the association colloids comprised in the pharmaceutical compositions according to the present disclosure have a narrow particle size distribution as indicated by the SPAN and PI. A narrow particle size distribution in a pharmaceutical composition enhances the product quality, performance, and consistency.

Association colloids of uniform size dissolve at a consistent rate, ensuring predictable and enhanced drug liberation after administration. Narrow distribution minimizes the presence of overly large or small particles, which could dissolve too slowly or too quickly.

A narrow particle size distribution also helps achieve uniformity in the composition, ensuring each dosage unit, such as each prefilled syringe, contains an accurate and consistent amount of phytonadione. This uniformity is critical for therapeutic efficacy and regulatory compliance.

The compositions according to the present disclosure with narrow particle size distributions allow for precise control of drug release profiles.

Uniform particle size also minimizes variability in drug dissolution and absorption, which can help avoid unintended spikes in drug concentration (Cₘₐₓ) that might lead to side effects or toxicity.

Independently, a small particle size and a narrow distribution improves the manufacturing processes such as mixing and sterile filtration, by reducing the likelihood of segregation and agglomeration.

Furthermore, the narrow particle size distribution results in a more appealing appearance of the product and easy, visual inspections, and better patient acceptability.

A narrow particle size distribution enhances the pharmaceutical composition's performance, quality, and manufacturability.

According to a preferred embodiment of the present disclosure, the pharmaceutical composition further comprises a buffering system. According to another preferred embodiment of the present disclosure, the pharmaceutical composition comprises an acetate buffer system, such as a mixture of sodium acetate and acetic acid.

According to a preferred embodiment of the present disclosure, the pharmaceutical composition comprises an acetate buffer system in a concentration in the range of from about 0.1 mg/ml to about 1.0 mg/ml. According to another preferred embodiment of the present disclosure, the pharmaceutical composition comprises an acetate buffer system in a concentration in the range of from about 0.2 mg/ml to about 0.8 mg/ml. According to another preferred embodiment of the present disclosure, the pharmaceutical composition comprises an acetate buffer system in a concentration in the range of from about 0.2 mg/ml to about 0.6 mg/ml. According to a further preferred embodiment of the present disclosure, the pharmaceutical composition comprises an acetate buffer system in a concentration in the range of from about 0.3 mg/ml to about 0.6 mg/ml.

According to another preferred embodiment of the present disclosure, the pharmaceutical composition comprises an acetate buffer system in a concentration of about 0.1 mg/ml or more. According to another preferred embodiment of the present disclosure, the pharmaceutical composition comprises an acetate buffer system in a concentration of about 0.2 mg/ml or more. According to another preferred embodiment of the present disclosure, the pharmaceutical composition comprises an acetate buffer system in a concentration of about 0.25 mg/ml or more. According to a further preferred embodiment of the present disclosure, the pharmaceutical composition comprises an acetate buffer system in a concentration of about 0.3 mg/ml or more.

According to another preferred embodiment of the present disclosure, the pharmaceutical composition comprises an acetate buffer system in a concentration of about 1.0 mg/ml or less. According to another preferred embodiment of the present disclosure, the pharmaceutical composition comprises an acetate buffer system in a concentration of about 0.8 mg/ml or less. According to another preferred embodiment of the present disclosure, the pharmaceutical composition comprises an acetate buffer system in a concentration of about 0.6 mg/ml or less. According to a further preferred embodiment of the present disclosure, the pharmaceutical composition comprises an acetate buffer system in a concentration of about 0.5 mg/ml or less.

It is understood that the presence of a buffer system may stabilize the pH of the pharmaceutical composition thus improving the shelf-life.

According to another preferred embodiment of the present disclosure, the pharmaceutical composition has a pH in the range of from about 3.0 to about 6.0. According to another preferred embodiment of the present disclosure, the pharmaceutical composition has a pH in the range of from about 4.0 to about 6.0. According to another preferred embodiment of the present disclosure, the pharmaceutical composition has a pH in the range of from about 4.5 to about 5.5. According to another preferred embodiment of the present disclosure, the pharmaceutical composition has a pH in the range of from about 5.0 to about 5.5. According to a further preferred embodiment of the present disclosure, the pharmaceutical composition has a pH in the range of from about 5.2 to about 5.4. According to a yet further preferred embodiment of the present disclosure, the pharmaceutical composition has a pH of about 5.3.

The inventors of the present disclosure have surprisingly found that the stability of phytonadione can be improved when the pH of the pharmaceutical composition is adjusted in the above range. If the above pH range is not met, phytonadione decomposes rapidly which reduces the shelf-life of the composition.

### The method of manufacturing

In a second aspect, the present disclosure is directed to a method of manufacturing the pharmaceutical composition according to the first aspect of the present disclosure comprising the steps of
a) mixing phytonadione and a non-ionic surfactant to provide a first mixture, and heating said first mixture to a first temperature T₁ to provide a first mixture having a temperature T₁, wherein T₁ is in the range of from about 50 °C to about 80 C;
b) dissolving an emulsifier in water for injection to provide a second mixture, and heating said second mixture to a second temperature T₂ to provide a second mixture having a temperature T₂, wherein T₂ is in the range of from about 50 °C to about 80 °C;
c) mixing said first mixture having a temperature T₁ obtained from step a) and said second mixture having a temperature T₂ obtained from step b) to provide a third mixture; and
d) cooling down said third mixture to a temperature T₃ in the range of from about 20 °C to about 30 °C to provide the pharmaceutical composition according to the first aspect of the present disclosure.

The inventors of the present disclosure have surprisingly found that the pharmaceutical composition according to the first aspect of the present disclosure can be advantageously prepared by a phase inversion emulsification method according to the second aspect of the present disclosure.

In one step of the method according to the method of the present disclosure (hereinafter "step a)"), a first mixture is prepared. For that, phytonadione and non-ionic surfactant are mixed and heated to a first temperature T₁ in the range of from about 50 °C to about 80 °C. Without wishing to be bound by theory, the polar heads of the non-ionic surfactant are placed at the heart of an association colloid, such as a micelle. The hydrophobic tails of the non-ionic surfactant are placed towards the oil phase comprising phytonadione. In other words, by heating the first mixture of non-ionic surfactant and phytonadione to a first temperature in the ranges indicated, a W/O emulsion is obtained. Therein, the polar head groups of the non-ionic surfactant, such as PS80, are completely dehydrated, and are placed at the core of the micelles thus formed. On the other hand, the hydrophobic tail groups are oriented towards the oil phase.

The temperature T₁ of the first mixture is advantageously chosen slightly above the phase inversion temperature.

According to a preferred embodiment of the method according to the second aspect of the present disclosure, T₁ is in the range of from about 60 °C to about 70 C. According to a further preferred embodiment of the present disclosure, T₁ is in the range of from about 60 °C to about 67 C. According to a further preferred embodiment of the method according to the second aspect of the present disclosure, T₁ is in the range of from about 60 °C to about 65 C.

If the temperature T₁ is below said lower limits, a W/O emulsion may not be obtained. If the temperature T₁ exceeds the above upper limits, phytonadione may degrade.

According to a preferred embodiment of the method according to the second aspect of the present disclosure, step a) is carried out in a time of about 10 min or less.

If the time is more than 10 min, phytonadione might be degraded. Therefore, it is advantageous to perform step a) in a time of 10 min or less.

According to a preferred embodiment of the method according to the second aspect of the present disclosure, the first mixture contains less than about 3 wt.-% of water based on the total weight of the first mixture.

According to another preferred embodiment of the method according to the second aspect of the present disclosure, the first mixture contains less than about 2 wt.-% of water based on the total weight of the first mixture.

According to a further preferred embodiment of the method according to the second aspect of the present disclosure, the first mixture contains less than about 1 wt.-% of water based on the total weight of the first mixture.

According to a yet further preferred embodiment of the method according to the second aspect of the present disclosure, the first mixture is substantially water-free.

The inventors of the present disclosure have found that the hydration of the hydrophilic heads of the non-ionic surfactant indirectly influences the curvature of the resulting association colloids by modifying the shape of non-ionic surfactant molecule. When the polar heads are highly hydrated, the shape is conic with the heads on the larger side, favouring a O/W emulsion. When the polar heads are dehydrated, the shape is still conic, but inverted, where the heads are on the narrow side, favouring a W/O emulsion.

According to another preferred embodiment of the method according to the second aspect of the present disclosure, the second mixture further comprises a buffering system. According to another preferred embodiment of the present disclosure, the second mixture comprises an acetate buffer system, such as a mixture of sodium acetate and acetic acid.

According to a preferred embodiment of the present disclosure, the second mixture comprises an acetate buffer system in a concentration in the range of from about 0.15 mg/ml to about 1.7 mg/ml. According to another preferred embodiment of the present disclosure, the second mixture comprises an acetate buffer system in a concentration in the range of from about 0.25 mg/ml to about 1.3 mg/ml. According to another preferred embodiment of the present disclosure, the second mixture comprises an acetate buffer system in a concentration in the range of from about 0.4 mg/ml to about 1.0 mg/ml. According to a further preferred embodiment of the present disclosure, the second mixture comprises an acetate buffer system in a concentration in the range of from about 0.5 mg/ml to about 0.8 mg/ml.

According to another preferred embodiment of the present disclosure, the second mixture comprises an acetate buffer system in a concentration of about 0.15 mg/ml or more. According to another preferred embodiment of the present disclosure, the second mixture comprises an acetate buffer system in a concentration of about 0.25 mg/ml or more. According to another preferred embodiment of the present disclosure, the second mixture comprises an acetate buffer system in a concentration of about 0.4 mg/ml or more. According to a further preferred embodiment of the present disclosure, the second mixture comprises an acetate buffer system in a concentration of about 0.5 mg/ml or more.

According to another preferred embodiment of the present disclosure, the second mixture comprises an acetate buffer system in a concentration of about 1.7 mg/ml or less. According to another preferred embodiment of the present disclosure, the second mixture comprises an acetate buffer system in a concentration of about 1.3 mg/ml or less. According to another preferred embodiment of the present disclosure, the second mixture comprises an acetate buffer system in a concentration of about 1.0 mg/ml or less. According to a further preferred embodiment of the present disclosure, the second mixture comprises an acetate buffer system in a concentration of about 0.8 mg/ml or less.

In a further step of the method according to the second aspect of the present disclosure (hereinafter referred to as step b), an emulsifier is dissolved in water for injection to provide a second mixture, and said second mixture is heated to a second temperature T₂ to provide a second mixture having a temperature T₂, wherein T₂ is in the range of from about 50 °C to about 80 °C.

The second mixture is an aqueous solution of an emulsifier, and optionally a buffering system.

According to a preferred embodiment of the method according to the second aspect of the present disclosure, T₂ is in the range of from about 60 °C to about 70 C.

According to a further preferred embodiment of the method according to the second aspect of the present disclosure, T₂ is in the range of from about 60 °C to about 65 C.

The second temperature T₂ of the second mixture is not particularly limited. However, it is advantageous that the second temperature T₂ is similar to the first temperature T₁ to allow for a controlled cooling process.

According to another preferred embodiment of the method according to the second aspect of the present disclosure, the difference between T₁ and T₂ is about 10 °C or less. According to another preferred embodiment of the method according to the second aspect of the present disclosure, the difference between T₁ and T₂ is about 5 °C or less. According to a further preferred embodiment of the method according to the second aspect of the present disclosure, the difference between T₁ and T₂ is about 3 °C or less. According to a yet further preferred embodiment of the method according to the second aspect of the present disclosure, T₁ and T₂ are about the same.

It is understood that the order of step a) and step b) are not particularly limited. According to a preferred embodiment of the present disclosure, step b) is performed before step a). According to another preferred embodiment of the second aspect of the present disclosure, step a) and step b) are performed at the same time.

In a subsequent step of the method according to the method of the second aspect of the present disclosure (hereinafter referred to as "step c)"), the first mixture having a temperature T₁ obtained from step a) and said second mixture having a temperature T₂ obtained from step b) are mixed to provide a third mixture.

Without wishing to be bound by theory, a continuous system is obtained. In that, water molecules start to hydrate the hydrophilic heads of the non-ionic surfactant, giving the non-ionic surfactant molecules, such as PS80, a straight shape. Thus, the non-ionic surfactants form large association colloids with the hydrophilic heads pointing to the surrounding water, and the lipophilic tails pointing to each other thereby forming a hydrophobic part where phytonadione can be stored. Also, the emulsifier may attach to the hydrophilic heads of the non-ionic surfactants to stabilize the association colloids.

According to a preferred embodiment of the method according to the second aspect of the present disclosure, step c) is performed at a temperature T₄, wherein T₄ is in the range between T₁ and T₂.

In other words, it is understood that step c) is performed at a temperature in the range between the first mixture's temperature T₁ to the second mixture's temperature T₂.

According to a preferred embodiment of the method according to the second aspect of the present disclosure, step c) further comprises adjusting the pH of the third mixture to a pH in the range of from about 5.0 to about 5.3. According to a further preferred embodiment of the method according to the second aspect of the present disclosure, step c) further comprises adjusting the pH of the third mixture to a pH of about 5.1.

Adjusting the pH in the above given range reduces the injection site adverse effects and further helps to stabilize the pharmaceutical composition, in particular the emulsion state of the pharmaceutical composition.

In a subsequent step of the method according to the second aspect of the present disclosure, said third mixture is cooled to a third temperature T₃ in the range of from about 20 °C to about 30 °C to provide the pharmaceutical composition according to the first aspect of the present disclosure.

When cooling down to the third temperature T₃, the solubility of the non-ionic surfactant, such as PS80, becomes greater towards water and the hydration degree of polar heads increases. The bicontinuous system breaks down to form O/W emulsion association colloids, preferably micelles, as the polar heads are being placed at the water-oil interface and the hydrophobic tails are on the inside of said association colloid.

Without wishing to be bound by theory, the polar heads of the non-ionic surfactant are completely hydrated, giving the molecule a cone shape. The resulting association colloids, such as micelles, have a round shape with the polar head of the non-ionic surfactant, such as PS80, pointing towards the water and the lipophilic tails point to the inside of the association colloids thereby forming a lipophilic space where phytonadione can be stored.

According to a preferred embodiment of the method according to the second aspect of the present disclosure, step d) further comprises a step of sterilizing the pharmaceutical composition.

According to a further preferred embodiment of the method according to the second aspect of the present disclosure, sterilizing comprises sterile filtration or terminal sterilization, preferably sterile filtration.

The inventors of the present disclosure have surprisingly found that the pharmaceutical composition according to the present disclosure is not affected by sterilization conditions. In particular, the compositions may advantageously be sterilized by filtration (see example 2) due to the small particle size of the association colloids and low SPAN and PI.

### The pharmaceutical composition prepared by a method of the present disclosure

In a third aspect, the present disclosure relates to a pharmaceutical composition obtained by the method according to the second aspect of the present disclosure.

### The container

According to a fourth aspect, the present disclosure relates to a container comprising the pharmaceutical composition according to the first aspect of the present disclosure or the pharmaceutical composition according to the third aspect of the present disclosure.

According to a preferred embodiment, the container comprising the pharmaceutical composition according to the fourth aspect of the present disclosure is a syringe.

According to a preferred embodiment, the container comprising the pharmaceutical composition according to the fourth aspect of the present disclosure is a glass syringe.

The inventors of the present disclosure have surprisingly found that the pharmaceutical composition according to the present disclosure can be store in prefilled syringes. Still, the compositions remain stable and phytonadione is stable upon storage and resistant to light.

According to another preferred embodiment of the present disclosure, the container according to the fourth aspect of the present disclosure is enclosed in a tertiary packaging. According to a further preferred embodiment of the present disclosure, the tertiary packaging is a carton box or an aluminium

### Definitions

As used herein, the term "association colloid" is a type of colloidal system formed by the self-assembly or aggregation of amphiphilic molecules (e.g., surfactants, block copolymers, or lipids) in a solvent, typically water, above a critical concentration threshold. These amphiphilic molecules spontaneously organize into distinct nanoscale structures, such as micelles, vesicles, or bilayers, driven by hydrophobic and hydrophilic interactions. The resulting colloidal entities are characterized by a dispersed phase where the aggregated structures maintain dynamic equilibrium with individual molecules in the continuous phase, providing unique physicochemical properties suitable for diverse industrial, pharmaceutical, or chemical applications. According to a preferred embodiment of the present disclosure, an association colloid is a micelle. In other words, the pharmaceutical composition according to the present disclosure comprises a plurality of micelles.

As used herein, the term "assay" of a compound (such as phytonadione, or any impurity) refers to the amount or level of the respective compound as determined by a method as disclosed herein, unless indicated otherwise.

The terms "phytonadione", "phytomenadione", "phylloquinone", "(E)-phytonadione", "vitamin K₁", and "2-Methyl-3-[(2*E*,7*R*,11*R*)-3,7,11,15-tetramethyl-2-hexadecen-1-yl]-1,4-naphthalenedione" are used interchangeably.

Phytonadione is also known under CAS number 84-80-0 and has the following chemical structure:

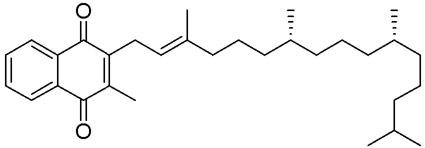

It is understood that phytonadione is sensitive to degradation, e.g. by heat, by oxidation, and/or by light. The most common degradation products of phytonadione are 1,2-quinone-methide, hydroperoxy-phytonadione, hydroxy-phytonadione, trans-epoxy-phytonadione, and phytonadione chromenol.

As used herein, 1,2-quinone-methide (CAS number 572-96-3) refers to a compound having the chemical structure:

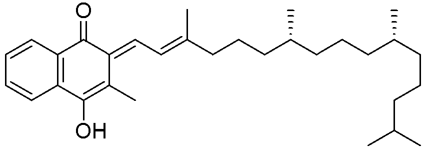

As used herein, hydroperoxy-phytonadione (CAS number 2241755-02-0) refers to a compound having the chemical structure:

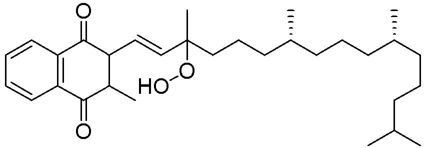

As used herein, hydroxy-phytonadione (CAS number 15576-40-6) refers to a compound having the chemical structure:

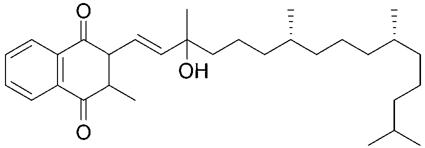

As used herein, trans-epoxy-phytonadione (CAS number 1588773-08-3) refers to a compound having the chemical structure:

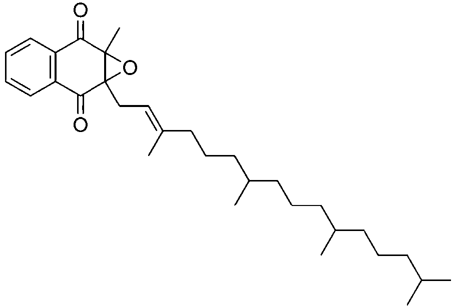

As used herein, phytonadione chromenol (CAS number 34044-00-3) refers to a compound having the chemical structure:

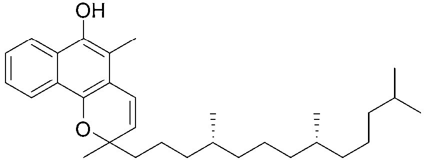

As used herein, the term "polysorbate" refers to a class of non-ionic surfactants. Chemically, polysorbates are derived from polyethoxylated sugar alcohol sorbitan, and they are modified by adding fatty acids. Mechanistically, polysorbates are used to help ingredients mix more easily and remain stable, especially when combining oil- and waterbased substances that typically do not blend well.

A polysorbate has the following general chemical structure: 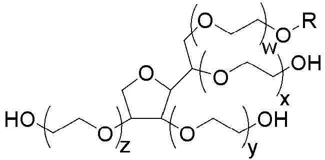 wherein each of w, x, y, and z is an integer in the range of from 0 to 20, and wherein the sum of w + x + y + z is preferably 20, and wherein R is a fatty acid group, such as laurate, palmitate, stearate, or oleate.

There are several types of polysorbates, each identified by a number (e.g., Polysorbate 20, 40, 60, 80), which indicates the type of fatty acid used in their synthesis.

For example, polysorbate 20 refers to a polysorbate, wherein R is laureate. As used herein, polysorbate 20 may be abbreviated as "PS20".

Polysorbate 40 refers to a polysorbate, wherein R is palmitate. As used herein, polysorbate 40 may be abbreviated as "PS40".

Polysorbate 60 refers to a polysorbate, wherein R is stearate. As used herein, polysorbate 60 may be abbreviated as "PS60".

Polysorbate 80 refers to a polysorbate, wherein R is oleate.

Polysorbate 80 is also known under CAS number 9005-65-6, or as *"polyethoxylene (2) sorbitan monooleate".* Polysorbate 80 is commercially available under brand names "Kolliphor PS 80", "Tween 80", "Montax 80", "Alkest 80", "PS 80", or "Kotilen-80". As used herein, polysorbate 80 may be abbreviated as "PS80".

As used herein, the term "mean particle size", or "Z-average" is the average hydrodynamic particle diameter. As used herein, the mean particle size is determined by dynamic light scattering, preferably by dynamic light scattering on a Malvern Zeta-sizer instrument.

As used herein, the terms "standard deviation of the particle size distribution" and "standard deviation of the particle size distribution (Z-average)" are used interchangeably and refer to the relative standard deviation of the mean particle size (average hydrodynamic particle diameter; Z-average). As used herein, the standard deviation of the particle size distribution is determined by dynamic light scattering, preferably by dynamic light scattering on a Malvern Zeta-sizer instrument.
As used herein, the "Dᵢ₁₀", "Dᵢ₅₀", and "Dᵢ₉₀" represent specific points in the intensity-based particle size distribution. As used herein, each of the "Dᵢ₁₀", "Dᵢ₅₀", and "Dᵢ₉₀" is determined by dynamic light scattering, preferably by dynamic light scattering on a Malvern Zeta-sizer instrument. In other words, for determining each of "Dᵢ₁₀", "Dᵢ₅₀", and "Dᵢ₉₀", a sample of a pharmaceutical composition according to the present disclosure is submitted to a light scattering method and the intensity-based particle size distribution is determined from the recorded signals.

As used herein, the "Dᵢ₁₀" is the intensity-based particle size at which 10 % of the total light scattering intensity comes from particles having diameter smaller than said Dᵢ₁₀. In other words, the Dᵢ₁₀ characterizes the smaller end of the particle size distribution, representing the particle diameter where particles smaller than Dᵢ₁₀ contribute 10 % of the total light scattering intensity. In yet other words, the Dᵢ₁₀ is the particle diameter below which 10 % of the total scattering intensity occurs. On the other hand, 90 % of the total light scattering intensity comes from particles having a diameter larger than or equal to said Dᵢ₁₀.

As used herein, the "Dᵢ₅₀" is the intensity-based median particle size at which 50 % of the total light scattering intensity comes from particles having a smaller diameter than said Dᵢ₅₀. In other words, Dᵢ₅₀ represents the middle point in the intensity-weighted distribution, often referred to as the median particle size representing the diameter where smaller than Dᵢ₅₀ contribute 50 % of the total light scattering intensity. In yet other words, the Dᵢ₅₀ is the particle diameter below which 50 % of the total scattering intensity occurs. On the other hand, 50 % of the total light scattering intensity comes from particles having a diameter larger than or equal to said Dᵢ₅₀.

As used herein, the "Dᵢ₉₀" is the intensity-based particle size at which 90 % of the total light scattering intensity comes from particles having diameter smaller than said Dᵢ₉₀. In other words, the Dᵢ₉₀ characterizes the larger end of the particle size distribution, representing the particle diameter where particles smaller than Dᵢ₉₀ contribute 90 % of the total light scattering intensity. In yet other words, the Dᵢ₉₀ is the particle diameter below which 90 % of the total scattering intensity occurs. On the other hand, 10 % of the total light scattering intensity comes from particles having a diameter larger than or equal to said Dᵢ₉₀.

As used herein, the term "SPAN" is calculated according to the formula $\frac{{D}_{i 90} - {D}_{i 10}}{{D}_{i 50}} .$

Thus, it is understood that the SPAN characterizes the width of the particle size distribution.

As used herein, the term "room temperature" refers to a temperature in the range of from about 20 °C to about 30 °C, preferably about 25 °C.

As used herein, the term "macroemulsion" refers to an emulsion containing particles having a particle size in the range of from about 1 µm to about 100 µm. Moreover, a macroemulsion may show a high polydispersity index (PI), has a turbid appearance and high viscosity. As such, a macroemulsion is thermodynamically unstable, and weakly kinetically stable.

As used herein, the term "nanoemulsion" refers to an emulsion containing particles having a particle size in the range of from about 20 nm to about 500 nm. Moreover, a nanoemulsion may show a low polydispersity index (PI), has a clear to slightly turbid appearance and moderate viscosity. As such, a nanoemulsion is thermodynamically unstable, but kinetically stable.

As used herein, the term "microemulsion" refers to an emulsion containing particles having a particle size in the range of from about 10 nm to about 100 nm. Moreover, a microemulsion may show a very low polydispersity index (PI), has a clear appearance and low, water-like viscosity. As such, a microemulsion is thermodynamically stable.

As used herein, the term "polydispersity index" is abbreviated as "PI" and defined as $PI = {\left(\frac{standard deviation of the mean particle size \left(Z-average\right)}{mean particle size}\right)}^{2}$

It is understood that a PI close to 0 suggests a narrow, nearly uniform size distribution (monodisperse). On the other hand, PI values above 0.1 generally indicate a broader size distribution (polydisperse), which might affect properties like stability, reactivity, or optical characteristics of the particle system.

As used herein, the "viscosity" is determined by measurement on a calibrated viscometer (e.g., Brookfield DVNext) at 25 °C (for both calibration measurements and sample measurements).

As used herein, the term "about" in connection with a numerical values refers to normal deviations of said numerical value. It is to be understood that the term "about" can mean a deviation of ± 10 %, preferably ± 5 %, more preferably ± 2.5 % of said numeric value as indicated.

### Phase transition temperature

As used herein, the terms "phase inversion temperature" (which may be abbreviated as "PIT"), "phase transition temperature", "critical micelle temperature" and "Krafft temperature" are used interchangeably. As used herein, the terms "phase inversion temperature" refers to the temperature at which a non-ionic surfactant in water undergoes a change from being dissolved in water as individual molecules to forming micelles. In other words, the "phase inversion temperature" marks the point where the formation of micelles becomes energetically favourable.

According to one embodiment, the phase inversion temperature is determined by differential scanning calorimetry. According to another embodiment, the phase transition temperature is determined according to method (1):

### Method (1)

In a first measurement, a solution of non-ionic surfactant in water is provided in a transparent container at 25 °C, such as a cuvette or glass vial, wherein the concentration of the non-ionic surfactant is above the critical micelle concentration (CMC) of said non-ionic surfactant. Then, the temperature of the solution of non-ionic surfactant in water is gradually increased, e.g. at a rate of 0.5 °C/min to 1.0 °C/min, while monitoring the turbidity of said solution of visually or with an instrument, such as a spectrometer. The phase transition temperature is determined the temperature is identified as the temperature at which i) the solution first becomes visibly cloudy when the turbidity of said solution is monitored visually, or ii) the absorbance of said solution suddenly increases, when the turbidity of said solution is monitored with an instrument, such as a spectrometer. It is preferable to monitor the turbidity of the solution of non-ionic surfactant in water in a spectrometer.

Optionally, in a second measurement, the phase transition temperature may be confirmed by heating a solution of non-ionic surfactant in water, wherein the concentration of the non-ionic surfactant is above the critical micelle concentration (CMC) of said non-ionic surfactant, to a temperature of 90 °C and subsequently cooling, e.g. at a rate of 5 °C/min to 1.0 °C/min, while monitoring the turbidity of said solution of visually or with an instrument, such as a spectrometer. The phase transition temperature is determined the temperature is identified as the temperature at which i) the solution first becomes visibly clear when the turbidity of said solution is monitored visually, or ii) the absorbance of said solution suddenly decreases, when the turbidity of said solution is monitored with an instrument, such as a spectrometer. The phase transition temperature is determined as the mean value of the first and second measurements.

It is preferable to monitor the turbidity of the solution of non-ionic surfactant in water in a spectrometer.

### Assay of phytonadione

As used herein, the "amount of phytonadione" contained in a pharmaceutical composition according to the present disclosure is determined by HPLC according to the phytonadione USP Monograph (official as of 1 May 2020) or the phytonadione injectable emulsion USP Monograph (official as of 1 May 2018).

According to an alternative embodiment, the "amount of phytonadione" contained in a pharmaceutical composition according to the present disclosure is determined by HPLC analysis combined with UV detection referred to as "method (2)":

### Method (2):

HPLC analysis can be performed on an Agilent HPLC system equipped with DAD or VWD.

For analysis of the pharmaceutical composition, 1.0 ml of pharmaceutical composition is withdrawn and diluted to a final volume of 20 ml using a mixture of anhydrous ethanol and H₂O in a ratio of 95:5 (v/v) to provide an analysis solution. No further sample preparation steps are needed other than the dilution.

For the mobile phase, a mixture of anhydrous ethanol and H₂O in a ratio of 95:5 (v/v) is used with isocratic profile at a flow rate of 0.7 mL/min.

The analysis solution is then subjected to HPLC on a column "µBondapak C18 250 x 4,6 mm, 10 µm", with a flow rate of 0.7 mL/min, whereas the column temperature is maintained at room temperature. Phytonadione was detected at a wavelength of 254 nm. The amount of phytonadione is determined by external calibration using phytonadione USP standard.

### Determination of phytonadione degradation products

As used herein, the "amount of phytonadione degradation products", such as the "amount of hydroperoxy-phytonadione", the "amount of hydroxy-phytonadione", and the "amount of trans-epoxy-phytonadione", contained in a pharmaceutical composition according to the present disclosure is determined by HPLC according to the USP Monograph phytonadione degradation products in Phytonadione Injectable Emulsion USP (official as of 1 May 2018) or according to method (3).

According to an alternative embodiment, the "amount of phytonadione degradation products", such as the "amount of hydroperoxy-phytonadione", the "amount of hydroxy-phytonadione", and the "amount of trans-epoxy-phytonadione" contained in a pharmaceutical composition according to the present disclosure is determined by HPLC analysis combined with UV detection referred to as "method (3)":

### Method (3):

HPLC analysis can be performed on an Agilent HPLC system equipped with DAD.

For analysis of the pharmaceutical composition, 5.0 ml of pharmaceutical composition is withdrawn and diluted to a final volume of 10 ml using acetonitrile to provide an analysis solution. No further sample preparation steps are needed other than the dilution.

For the mobile phase, a mixture of acetonitrile and an aqueous 2 mM ammonium acetate solution in a ratio of 95:5 (v/v) is used with isocratic profile at a flow rate of 1.5 mL/min.

The analysis solution is then subjected to HPLC on a column "Poroshell EC-C18 250 x 4.6 mm, 2.7 µm", with a flow rate of 1.5 mL/min, whereas the column temperature is maintained at room temperature. Phytonadione degradation products were detected at a wavelength of 245 nm. The amount of phytonadione degradation products is determined by external calibration using phytonadione USP standard and specified degradation products response factors determined during method validation.

### Examples

To further illustrate the present disclosure, the following examples are provided. It is to be understood that these examples are provided for illustrative purposes and are not to be construed as limiting the scope of the present disclosure.

### Example 1: Preparation of pharmaceutical composition

The following pharmaceutical compositions were prepared according to the following procedure. The final composition of the pharmaceutical compositions is indicated in Table 1.

PS80 and phytonadione are mixed at a ratio as indicated in Table 1 under stirring and heated to a first temperature T₁ as indicated in Table 1 to provide a first mixture having a temperature T₁. In this, no water is added.

In parallel, water for injection (60 % of final batch volume) is heated to a second temperature T₂ and dissolved oxygen is removed by sparging with nitrogen. Propylene glycol, sodium acetate, and glacial acetic acid are then added to the water to provide a second mixture. The temperature is maintained at said second temperature T₂ under nitrogen blanketing as indicated in Table 1.

Then, the first mixture having a temperature T₁ and the second mixture having a temperature T₂ were mixed under gentle stirring to provide a third mixture, which was then allowed to cool down to room temperature to obtain a pharmaceutical composition.

**Table 1 Composition of pharmaceutical composition and temperatures**

| **No.** | **Composition** | | | | | **Temperatures** | |
|---|---|---|---|---|---|---|---|
| | Phytonadione [mg/mL] | PS80 [mg/mL] | propylene glycol [mg/mL] | Na acetate [µg/mL] | glacial acetic acid | first temperature T₁ [°C] | second temperature T₂ [°C] |
| 1 | 2 | 30 | 10.4 | 0.17 | q.s. to pH 5.1 | 60-65 | 60-65 |
| 2 | 2 | 12 | 10.4 | 0.17 | | 60-65 | 60-65 |
| 3 | 2 | 20 | 10.4 | 0.17 | | 45-50 | 45-50 |
| 4 | 2 | 20 | 10.4 | 0.17 | | 75-80 | 75-80 |
| 5 | 2 | 20 | 10.4 | 0.17 | | 60-65 | 60-65 |

The compositions 1 to 5 thus obtained were subjected to particle size distribution analysis by DLS on a Malvern Zetasizer instrument. The results are summarized in Table 2.

**Table 2 Summary of particle size distribution analysis by DLS**

| **No.** | Ratio PS80/phytonadione | Mean [nm] | Dᵢ₁₀ [nm] | Dᵢ₅₀ [nm] | Dᵢ₉₀ [nm] | SPAN | PI |
|---|---|---|---|---|---|---|---|
| 5 | 10 | 13.15 | 9.61 | 13.40 | 18.93 | 0.70 | 0.04 |
| 1 | 15 | 11.65 | 8.46 | 11.94 | 16.98 | 0.71 | 0.03 |
| 2 | 6 | 14.40 | 10.49 | 14.69 | 20.45 | 0.68 | 0.02 |
| 3 | 10 | 13.90 | 9.02 | 14.54 | 24.20 | 1.04 | 0.15 |
| 4 | 10 | 13.08 | 9.51 | 13.40 | 19.22 | 0.72 | 0.04 |

It is understood that by heating the mixture of PS80 and phytonadione to a first temperature of about 60 °C to about 65 °C, a W/O emulsion is obtained, wherein the polar head groups of PS80 are completely dehydrated and are placed at the core of the micelles thus formed. On the other hand, the hydrophobic tail groups of PS80 are oriented towards the oil phase.

Upon mixing said W/O emulsion with the second composition having a temperature T₂, a bicontinuous system is obtained. Upon cooling down to 25°C, the solubility of PS80 becomes greater towards water and the hydration degree of polar heads increases.

Thus, the bicontinuous system breaks down to form O/W emulsion micelles. In that, the micelles are inverted, and the polar heads are being placed at the water interface and the hydrophobic tails are oriented towards the core of the micelles. Thereby, the phytonadione is incorporated into the hydrophobic part of the micelles.

The analysis of the particle size revealed that preparing a pharmaceutical composition at a temperature below the phase transition temperature, i.e. below 60 °C (formulation No. 3), does not ensure the formation of a monodisperse particle size distribution. Instead, composition No. 3 contains micelles of varying size as can be concluded from the SPAN of 1.04 and the PI being 0.15. This is explained by the formation of a secondary population of micelles having a diameter in the range of from 1 µm to 100 µm. Because said secondary population is not in the same size range than the main population, the width of the distribution is increased. In other words, a bimodal particle size distribution was observed. This indicates that the emulsification process was not satisfactory; a pharmaceutical composition should comprise only one population of micelles.

### Example 2: Sterilization

A pharmaceutical composition was prepared in accordance with example 1, composition No. 5.

The resulting composition was sterilized as follows:

### a. Filtration

The composition was filtered through to redundant Sartorius Sartopore^{®} 2 Filter 0.45/0.2 µm and analyzed. The results are given in Table 3.

**Table 3 Influence of sterile filtration on compositions**

| | **Before filtration** | **After filtration** |
|---|---|---|
| Assay | 110.6 % | 111.6 % |
| pH | 5.3 | 5.3 |
| Assay hydroperoxy-phytonadione | 0.81 % | 0.89 % |
| Assay hydroxy-phytonadione | ND | ND |
| Assay transepoxy-phytonadione | 0.17 % | 0.17 % |
| Assay total impurities | 1.2 % | 1.2 % |

| | | |
|---|---|---|
| ND: not detected | | |

No impact was observed when using the double filtration with Sartopore^{®}2. All results met the acceptance criteria for Assay and pH. The reported degradation products indicate that no impurities were above the limit and no new impurity was detected after the filtration.

### b. Terminal sterilization

Apart from the double filtration system study, the impact of a terminal sterilization procedure was evaluated to investigate into the impact of heat on composition No. 5. For that, composition No. 5 was prepared in accordance with example 1 and filled into a glass syringe and subsequently sealed. Three cycles of terminal sterilization were used: 121 °C for 8 minutes, 121 °C for 15 minutes, and 121 °C for 20 minutes. The sterilized compositions were subsequently analyzed according to method (2) and method (3); results are summarized in Table 4.

**Table 4 Influence of terminal sterilization on compositions**

| | **Before sterilization** | **8 min @ 121 °C** | **15 min @ 121 °C** | **20 min @ 121 °C** |
|---|---|---|---|---|
| **Assay [%]** | 109.9 | 94.6 | 96.4 | 95.5 |
| **Assay hydroperoxyphytonadione [%]** | 0.29 | 0.27 | 0.25 | 0.23 |
| **Assay hydroxy-phytonadione [%]** | ND | <QL | <QL | <QL |
| **Assay transepoxy-phytonadione [%]** | 0.29 | 0.21 | 0.21 | 0.22 |
| **Assay total impurities [%]** | 0.9 | 0.8 | 0.8 | 0.7 |
| **Mean particle size [nm]** | 13 | 14 | 14 | 14 |
| **Dᵢ₅₀ [nm]** | 9.7 | 9.1 | 9.1 | 9.3 |
| **Dᵢ₅₀ [nm]** | 13.6 | 14.0 | 14.2 | 14.1 |
| **Dᵢ₉₀ [nm]** | 19.6 | 22.8 | 22.6 | 23.1 |
| **SPAN** | 0.7 | 1.0 | 0.9 | 1.0 |
| **PI** | 0.04 | 0.18 | 0.14 | 0.20 |

| | | | | |
|---|---|---|---|---|
| ND: not detected QL: quantification limit | | | | |

In all compositions that were subjected to terminal sterilization, the formation of precipitates was observed. Hence, the description of the terminally sterilized prefilled syringes does not respect the acceptance criteria as formation of particulate matter was observed.

Accordingly, also the amount of phytonadione in the composition after terminal sterilization is considerably reduced. Without wishing to be bound by theory, it may be assumed that the decreased amount of phytonadione may be due to precipitation. The decrease in hydroperoxy-phytonadione might be explained by its heat sensitivity and its transformation to hydroxy-phytonadione when heated.

Furthermore, the formation of a secondary population of micelles having a particle size in the range of from 1 µm to 100 µm was observed. In other words, the compositions may show a bimodal particle size distribution.

### Example 3: Study of photo- and oxygen sensitivity

The degradation from light and/or oxygen was evaluated by quantifying the oxidation product hydroperoxy-phytonadione that is an indicator for photodegradation and oxidation of phytonadione. The experiment was conducted under different conditions. Two different lamp sources were tested, a light emitting diode (LED, Table 5) and a high-pressure sodium lamp (HPS, Table 5). Both lamps were used to test different light intensities (100 and 1000 lux, respectively). The stability against oxidation was evaluated by preparing syringes with different % O₂ in their headspace: 5 % (v/v) or 21 % (v/v) (ambient air).

**Table 5 Influence of light exposure and headspace oxygen on compositions**

| | | **Assay of hydroperoxy-phytonadione (%)** | | | | | | | |
|---|---|---|---|---|---|---|---|---|---|
| Light source | | **HPS** | | | | **LED** | | | |
| % O₂ in headspace | | 5 % O₂ (v/v) | | 21 % O₂ (v/v) | | 5 % O₂ (v/v) | | 21 % O₂ (v/v) | |
| Exposition time (min) | | 0 | 120 | 0 | 120 | 0 | 120 | 0 | 120 |
| Light intensity | 100 lux | 0.8 | 0.9 | 1.0 | 1.2 | 0.8 | 0.9 | 1.0 | 1.2 |
| | 1000 lux | 0.8 | 1.2 | 1.0 | 1.5 | 0.7 | 1.3 | 1.0 | 1.6 |

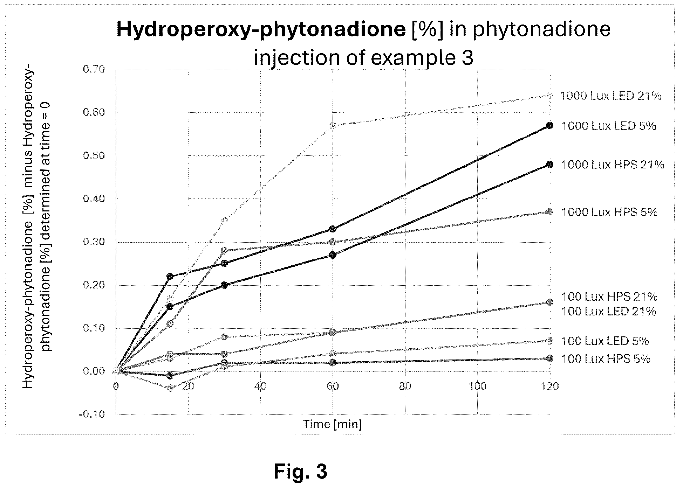

The comparison between the different lamp sources shows a higher degradation rate for the samples irradiated with the LED source that emits a broader range of wavelengths than the HPS source (Table 5 and Fig. 3).

Fig. 3 shows the levels of hydroperoxy-phytonadione determined by method (3) at different exposition times from which the levels of hydroperoxy-phytonadione determined by method (3) before exposition was subtracted. Thus, Fig. 3 shows that the levels of hydroperoxy-phytonadione in the composition only increases slightly.

The main factor influencing phytonadione degradation is light. No significant difference between nitrogen flushed samples and samples without nitrogen can be observed. The samples that were exposed to LED showed higher degradation rate because the LED emits a broader range of wavelengths than the HPS source (Table 5 and Fig. 3). The exposure to a higher light intensity (1000 lux) generated higher levels of hydroperoxy-phytonadione.

Phytonadione is less susceptible to photodegradation and/or oxidation when handled under light sources that emit only a specific range of wavelengths (e.g. HPS) with a maximum light intensity of 100 lux. At 100 lux, the hydroperoxy-phytonadione levels are stable.

Again, no significant difference between nitrogen flushed samples and samples without nitrogen can be observed, indicating solely a correlation between light exposure and the increase in degradation.

### Example 4: Characteristics of the microemulsion

The phytonadione injectable emulsion is characterized as a microemulsion. The micelles of the emulsion have a particles size of 13 nm. The viscosity of the emulsion was determined 1.1 cP that is close to water (1.0 cP). The microemulsions are thermodynamically stable.

### Example 5: Study of storage stability

A pharmaceutical composition was prepared in accordance with example 1, composition No. 5 and subsequently sterilized according to example 2. The phytonadione injectable emulsion was then evaluated in its stability in different examples.

### pH adjustment and stability

The stability of phytonadione injectable emulsions USP 1 mg/0.5 ml in syringes at different pH targets of 4.0, 4.5, 5.0, 5.5, and 6.0 was evaluated in stability chambers with different conditions. All samples of different pH targets were stored for 14 days at 25 °C/60 % RH and 30 °C/65 % RH. All samples were protected from light.

The stability was determined as indicated by the increase in total of degradation products. The most stable solutions with the lowest increase of degradation products were solution with a pH from 5.0 to 5.5.

### Stability study

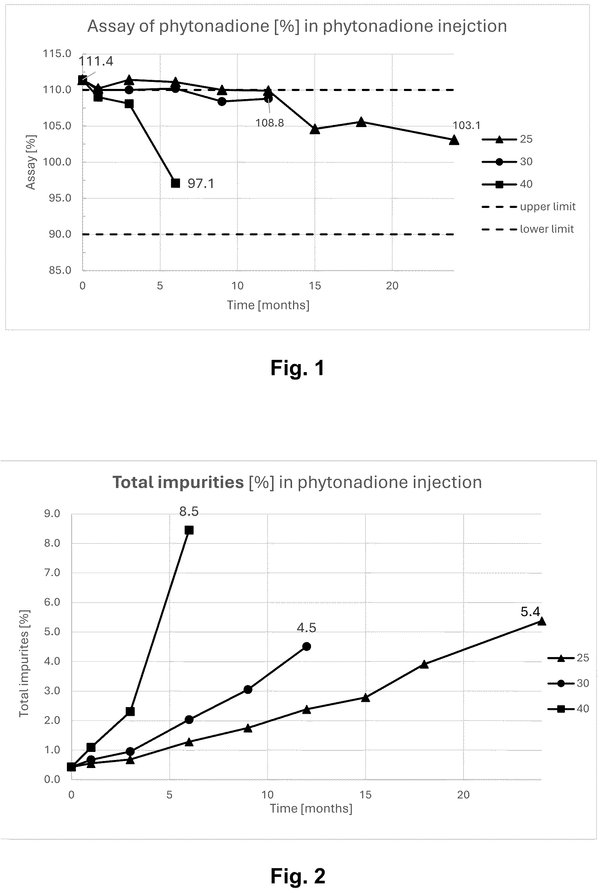

The formulation of the phytonadione injectable emulsion 1 mg/ 0.5 ml are filled into 1 ml syringes with a headspace of 4% residual oxygen and kept under three different storage conditions: long-term stability conditions at 25±2 °C / 60±5 % RH, intermediate conditions at 30±2 °C / 65±5 % RH, and accelerated stability conditions at 40±2 °C / 75±5 % RH. The syringes were stored in vertical position under exclusion of light and analyzed according to method (2) and method (3). The results are reported in Tables 6 to 8 and Figs. 1 and 2.

**Table 6 Phytonadione injectable emulsion under long-term conditions at 25±2 °C / 60±5% RH.**

| **Test / Timepoint (months)** | **0** | **1** | **3** | **6** | **9** | **12** | **15** | **24** |
|---|---|---|---|---|---|---|---|---|
| **Assay phytonadione [%]** | 111.4 | 110.2 | 111.4 | 111.1 | 110.0 | 109.9 | 105.6 | 103.1 |
| **pH** | 5.3 | 5.3 | 5.3 | 5.4 | 5.4 | 5.4 | 5.4 | 5.4 |
| **Assay hydroperoxy-phytonadione [%]** | 0.26 | 0.38 | 0.52 | 0.96 | 1.42 | 1.87 | 2.35 | 4.07 |
| **Assay hydroxy-phytonadione [%]** | ND | ND | ND | <QL | <QL | 0.07 | 0.11 | 0.39 |
| **Assay transepoxy-phytonadione [%]** | 0.17 | 0.17 | 0.16 | 0.17 | 0.16 | 0.18 | 0.19 | 0.23 |
| **Assay total impurities [%]** | 0.4 | 0.6 | 0.7 | 1.3 | 1.8 | 2.4 | 3.9 | 5.4 |

**Table 7 Phytonadione injectable emulsion under intermediate conditions at 30±2°C / 65±5% RH.**

| **Test / Timepoint (months)** | **0** | **1** | **3** | **6** | **9** | **12** |
|---|---|---|---|---|---|---|
| **Assay of phytonadione [%]** | 111.4 | 110.0 | 110.0 | 110.2 | 108.4 | 108.8 |
| **pH** | 5.3 | 5.3 | 5.3 | 5.4 | 5.4 | 5.4 |
| **Assay hydroperoxy-phytonadione [%]** | 0.26 | 0.49 | 0.71 | 1.56 | 2.44 | 3.35 |
| **Assay hydroxy-phytonadione [%]** | ND | ND | <QL | 0.04 | 0.11 | 0.23 |
| **Assay transepoxy-phytonadione [%]** | 0.17 | 0.18 | 0.15 | 0.18 | 0.17 | 0.20 |
| **Assay total impurities [%]** | 0.3 | 0.7 | 1.0 | 2.0 | 3.1 | 4.2 |

**Table 8 Phytonadione injectable emulsion under accelerated conditions at 40±2°C / 75±5% RH.**

| **Test / Timepoint (months)** | **0** | **1** | **3** | **6** |
|---|---|---|---|---|
| **Assay of phytonadione [%]** | 111.4 | 109.0 | 108.1 | 97.1 |
| **pH** | 5.3 | 5.4 | 5.4 | 5.7 |
| **Assay hydroperoxy-phytonadione [%]** | 0.26 | 0.81 | 1.9 | 5.5 |
| **Assay hydroxy-phytonadione [%]** | ND | <QL | 0.08 | 0.51 |
| **Assay transepoxy-phytonadione [%]** | 0.07 | 0.18 | 0.17 | 0.31 |
| **Assay total impurities [%]** | 0.33 | 1.09 | 2.30 | 8.45 |

No visible changes were found in any of the samples, a conforming yellow solution was present in all samples at different conditions and at all time points.

The pH values are within the acceptance criteria of a pH value of 3.5 to 7 for all samples at different conditions and at all time points. The pH values slightly increase over time. The suitable range of a pH ≤ 5.5, as established in the example *pH adjustment and stability,* is maintained in all samples except at the end of stability under accelerated conditions

The assay remains stable during 12 months under long term conditions. For intermediate conditions a loss in assay of 2.6 % after 12 months stability is observed. An even bigger assay loss of 14.3 % is observed for the accelerated condition after six months, indicating an increased degradation under increased heat conditions. The assay still respects the acceptance criteria (90.0 - 110.0%) in all stability conditions.

For all three conditions an increase in hydroperoxy-phytonadione is observed. All samples are within the accepted levels (NMT 3.0 %), except from the sample stored under accelerated conditions after six months that is above this limit.

Hydroxy-phytonadione was under the limit of quantitation for the sample under long-term conditions until month 12. For the sample stored under intermediate conditions the limit (NMT 0.2 %) was exceeded after 12 months to 0.23 %. The biggest increase, exceeding the accepted limit, was observed under accelerated conditions after six months to 0.51 %.

Transepoxy-phytonadione remained stable under long-term and intermediate conditions at all time points. In accelerated conditions, the epoxy impurity increased to 0.31% after six months, exceeding the accepted limit (NMT 0.5 %).

### Example 6: Comparison to Amphastar product

### Photostability under ICH guidelines

Furthermore, the phytonadione injectable emulsion 1 mg/0.5 ml was exposed to cool white fluorescent (1.2 Mlux×h) and near ultraviolet lights (200 W×h/m²) to evaluate the impact of light exposition as indicated by ICH guideline Q1B (Photostability Testing of New Drug Substance and Product). The injectable emulsion was compared to comparison product Amphastar, in their respective secondary and tertiary packaging.

The prefilled syringe according to the present disclosure and the Amphastar cartridge/vial stored in their respective secondary packaging (individual carton box) or the tertiary packing (marketing pack) were exposed to light. As dark controls, the different packaging types were wrapped in opaque foil, to block light exposition. The results are displayed in Tables 9 and 10.
The color of the solution changed in both samples from a clear pale-yellow solution in the dark control to a dark yellow solution, as expected through the degradation.

**Table 9 Results for photostability for the solution according to present disclosure and the Amphastar comparison product in their secondary packing.**

| | **example** | | **Amphastar** | |
|---|---|---|---|---|
| | **Dark control** | **Exposed individual box** | **Dark control** | **Exposed individual box** |
| **Assay of phytonadione [%]** | 104.9 | 74.0 | 107.5 | 75.0 |
| **Assay hydroperoxy-phytonadione [%]** | 0.8 | 1.5 | 0.7 | 6.9 |
| **Assay hydroxy-phytonadione [%]** | <QL | n.d. | <QL | 0.6 |
| **Assay transepoxy-phytonadione [%]** | 0.3 | 0.3 | ND | ND |
| **Assay total unspecified impurities [%]** | 0.0 | 9.9 | 0.3 | 10.6 |
| **Assay total impurities [%]** | 1.0 | 14.3 | 1.0 | 18.1 |

| | | | | |
|---|---|---|---|---|
| n.d.: not determined | | | | |

The control samples both meet the acceptance criteria (90.0 - 110.0 % Phytonadione). The exposed samples, both the PFS example and the Amphastar vial, do not fulfil this requirement.

The total impurities increased to 14.1 % after exposure in the present example and are lower compared to 18.1 % for the Amphastar product. The value for hydroperoxy-phytonadione in the example control sample is 0.8% and 1.5% for the exposed sample. Both values meet the acceptance criteria of 5.0%. The comparison product Amphastar almost ten-folds the hydroperoxy-phytonadione level in the exposed sample and does not meet the acceptance criteria of 5 %.

**Table 10 Results for photostability for the solution according to present disclosure and the Amphastar comparison product in their tertiary packing.**

| | **example** | | **Amphastar** | |
|---|---|---|---|---|
| | **Dark control** | **Exposed marketing pack** | **Dark control** | **Exposed marketing pack** |
| **Assay of phytonadione [%]** | 104.2 | 101.1 | 105.0 | 91.3 |
| **Assay hydroperoxy-phytonadione [%]** | 0.8 | 1.7 | 0.5 | 2.8 |
| **Assay hydroxy-phytonadione [%]** | <QL | <QL | <QL | 0.5 |
| **Assay transepoxy-phytonadione [%]** | 0.3 | 0.3 | <QL | 0.1 |
| **Assay total unspecified impurities [%]** | 0.0 | 0.8 | 0.3 | 5.2 |
| **Assay total impurities [%]** | 1.0 | 2.8 | 0.8 | 8.6 |

The inventive composition and the Amphastar comparison product met the acceptance criterion for phytonadione upon storage in the dark. After light exposition, the composition according to the present disclosure has a considerably higher stability than the amphastar product. The 3% assay loss in the composition according to the present disclosure is considered an acceptable change; the assay loss in the Amphastar product is 14%.

The Amphastar product shows higher levels of hydroperoxy-phytonadione and hydroxy-phytonadione upon light exposition. It does not meet the acceptance criteria for hydroxy-phytonadione levels under 0.2 %. The present example is within the acceptance criteria required for all specified impurities and results in 2.8 % total impurities compared to 8.6% total impurities in the Amphastar product.

### Comparison of physicochemical parameters

**Table 11 Comparative analysis of physicochemical parameters**

| | **Example batches 1 - 3** | | | **Amphastar batches 1 - 3** | | |
|---|---|---|---|---|---|---|
| **Assay of phytonadione [%]** | 106.4 | 106.0 | 105.6 | 107.3 | 106.3 | 106.6 |
| **Assay hydroperoxy-phytonadione [%]** | 0.9 | 0.6 | 0.8 | 0.4 | 0.5 | 0.5 |
| **Assay hydroxy-phytonadione [%]** | <QL | <QL | <QL | <QL | <QL | <QL |
| **Assay transepoxy-phytonadione [%]** | 0.3 | 0.3 | 0.3 | 0.1 | 0.1 | 0.1 |
| **Assay total impurities [%]** | 1.1 | 0.9 | 1.1 | 14.7 | 14.7 | 14.8 |
| **pH** | 5.6 | 5.6 | 5.4 | 5.0 | 5.1 | 5.0 |
| **Mean particle size [nm]** | 13.07 | 13.13 | 12.97 | 13.10 | 13.44 | 14.05 |
| **Dᵢ₁₀ [nm]** | 9.3 | 9.5 | 9.3 | 9.1 | 8.9 | 9.0 |
| **Dᵢ₅₀ [nm]** | 13.5 | 13.5 | 13.3 | 13.8 | 13.9 | 13.6 |
| **Dᵢ₉₀ [nm]** | 19.7 | 19.3 | 19.3 | 20.8 | 22.4 | 1201.0 |
| **SPAN** | 0.77 | 0.73 | 0.75 | 0.85 | 0.97 | 87.40 |
| **PI** | 0.05 | 0.04 | 0.05 | 0.09 | 0.14 | 0.18 |
| **Viscosity** | 1.12 | 1.12 | 1.13 | 1.09 | 1.10 | 1.09 |
| **Osmolarity** | 311 | 307 | 310 | 312 | 312 | 312 |
| **Surface tension** | 37.36 | 31.10 | 32.28 | 34.75 | 34.55 | 38.29 |
| **Density** | 1.0007 | 1.0008 | 1.0008 | 1.0006 | 1.0007 | 1.0006 |

All known impurities were well below the specifications limit for both products. However, both products have great differences in their impurity profile, especially in the total degradation products that is in the present example at 0.9 to 1.1 %, compared to 14.7 to 14.8 % in the compared Amphastar product.

The pH values remained in the specification limit for all samples without great variation.

The distribution curves parameters (SPAN & Dᵢ₉₀) indicate that the Amphastar samples tested had a small portion of larger size particles, especially referring to batch 3 (column 3, Amphastar product) compared to the present example. The comparability study demonstrated a similar particle size distribution with a somewhat tighter emulsion homogeneity in the present example.

### Embodiments

1. A pharmaceutical composition comprising
   a) phytonadione,
   b) a non-ionic surfactant,
   c) an emulsifier; and
   d) water for injection, wherein
      the ratio $\frac{weight \left(non-ionic surfactant\right)}{weight \left(phytonadione\right)}$
      is in the range of from about 5 to about 20, and wherein
      the pharmaceutical composition comprises a plurality of association colloids having a mean particle size in the range of from about 10 nm to about 20 nm.
2. The pharmaceutical composition according to embodiment 1, wherein the mean particle size is in the range of from about 10 nm to about 15 nm.
3. The pharmaceutical composition according to embodiment 1 or 2, wherein the mean particle size is about 10 nm to about 13 nm.
4. The pharmaceutical composition according to any one of embodiments 1 to 3, wherein the mean particle size is about 13 nm.
5. The pharmaceutical composition according to any one of embodiments 1 to 4, wherein the non-ionic surfactant is a polysorbate.
6. The pharmaceutical composition according to any one of embodiments 1 to 5, wherein the non-ionic surfactant is polysorbate 80 (PS80).
7. The pharmaceutical composition according to any one of embodiments 1 to 6, wherein the pharmaceutical composition comprises phytonadione in a concentration of about 2 mg/mL.
8. The pharmaceutical composition according to any one of embodiments 1 to 7, wherein the ratio $\frac{weight \left(non-ionic surfactant\right)}{weight \left(phytonadione\right)}$ is in the range of from about 6 to about 15.
9. The pharmaceutical composition according to any one of embodiments 1 to 8, wherein the ratio $\frac{weight \left(non-ionic surfactant\right)}{weight \left(phytonadione\right)}$ is about 10.
10. The pharmaceutical composition according to any one of embodiments 1 to 9, wherein the phase inversion temperature is in the range of from about 50 °C to about 80 °C.
11. The pharmaceutical composition according to embodiment 10, wherein the phase inversion temperature is in the range of from about 60 °C to about 70 °C.
12. The pharmaceutical composition according to embodiment 10 or 11, wherein the phase inversion temperature is in the range of from about 60 °C to about 65 °C.
13. The pharmaceutical composition according to any one of embodiments 1 to 12, wherein the pharmaceutical composition comprises a plurality of association colloids having a SPAN of in the range of from about 0.6 to about 0.9.
14. The pharmaceutical composition according to embodiment 13, wherein the SPAN is in the range of from about 0.65 to about 0.8.
15. The pharmaceutical composition according to embodiment 13 or 14, wherein the SPAN is in the range of from about 0.65 to about 0.75.
16. The pharmaceutical composition according to any one of embodiments 13 to 15, wherein the SPAN is about 0.7.
17. The pharmaceutical composition according to any one of embodiments 1 to 16, wherein the pharmaceutical composition is in the form of a microemulsion.
18. The pharmaceutical composition according to any one of embodiments 1 to 17, wherein the pharmaceutical composition comprises a plurality of association colloids having a polydispersity index (PI) of less than 0.1.
19. A method of manufacturing the pharmaceutical composition according to any one of embodiments 1 to 18 comprising the steps of
   a) mixing phytonadione and a non-ionic surfactant to provide a first mixture, and heating said first mixture to a first temperature T₁ to provide a first mixture having a temperature T₁, wherein T₁ is in the range of from about 50 °C to about 80 C;
   b) dissolving an emulsifier in water for injection to provide a second mixture, and heating said second mixture to a second temperature T₂ to provide a second mixture having a temperature T₂, wherein T₂ is in the range of from about 50 °C to about 80 °C;
   c) mixing said first mixture having a temperature T₁ obtained from step a) and said second mixture having a temperature T₂ obtained from step b) to provide a third mixture; and
   d) cooling down said third mixture to a temperature T₃ in the range of from about 20 °C to about 30 °C to provide the pharmaceutical composition according to any one of embodiments 1 to 18.
20. The method according to embodiment 19, wherein step c) is performed at a temperature T₄, wherein T₄ is in the range between T₁ and T₂.
21. The method according to embodiment 19 or 20, wherein T₁ is in the range of from about 60 °C to about 70 C.
22. The method according to any one of embodiments 19 to 21, wherein T₁ is in the range of from about 60 °C to about 65 C.
23. The method according to any one of embodiment 19 to 22, wherein T₂ is in the range of from about 60 °C to about 70 C.
24. The method according to any one of embodiments 19 to 23, wherein T₂ is in the range of from about 60 °C to about 65 C.
25. The method according to any one of embodiments 19 to 24, wherein the difference between T₁ and T₂ is about 10 °C or less.
26. The method according to any one of embodiments 19 to 25, wherein the difference between T₁ and T₂ is about 5 °C or less.
27. The method according to any one of embodiments 19 to 26, wherein the difference between T₁ and T₂ is about 3 °C or less.
28. The method according to any one of embodiments 19 to 27, wherein T₁ and T₂ are about the same.
29. The method according to any one of embodiments 19 to 28, wherein step d) further comprises a step of sterilizing the pharmaceutical composition.
30. The method according to any one of embodiments 19 to 29, wherein sterilizing comprises sterile filtration or terminal sterilization.
31. The method according to any one of embodiments 19 to 30, wherein step a) is carried out in a time of about 10 min or less.
32. The method according to any one of embodiments 19 to 31, wherein step c) further comprises adjusting the pH of the third mixture to a pH in the range of from about 5.0 to about 5.3.
33. The method according to any one of embodiments 19 to 32, wherein step c) further comprises adjusting the pH of the third mixture to a pH of about 5.1.
34. The method according to any one of embodiments 19 to 33, wherein the first mixture contains less than about 3 wt.-% of water based on the total weight of the first mixture.
35. The method according to any one of embodiments 19 to 34, wherein the first mixture contains less than about 2 wt.-% of water based on the total weight of the first mixture.
36. The method according to any one of embodiments 19 to 35, wherein the first mixture contains less than about 1 wt.-% of water based on the total weight of the first mixture.
37. The method according to any one of embodiments 19 to 36, wherein the first mixture is substantially water-free.
38. A pharmaceutical composition obtained by the method according to any one of embodiments 19 to 37.
39. A container comprising the pharmaceutical composition according to any one of embodiments 1 to 18 or 38.
40. The container comprising the pharmaceutical composition according to embodiment 39, wherein the container is a syringe.
41. The container comprising the pharmaceutical composition according to embodiment 39 or 40, wherein the container is a glass syringe.

## Claims

1. A pharmaceutical composition comprising:
a) Phytonadione;
b) a non-ionic surfactant;
c) an emulsifier; and
d) water for injection, wherein the ratio $\frac{weight \left(non-ionic surfactant\right)}{weight \left(phytonadione\right)}$
is in the range of from about 5 to about 20, and wherein
the pharmaceutical composition comprises a plurality of association colloids having a mean particle size in the range of from about 10 nm to about 20 nm.

2. The pharmaceutical composition according to claim 1, wherein the mean particle size is in the range of from about 10 nm to about 15 nm, preferably wherein the mean particle size is in the range of from about 10 nm or about 13 nm, more preferably wherein the mean particle size is about 10 nm or about 13 nm.

3. The pharmaceutical composition according to claim 1 or 2, wherein the non-ionic surfactant is a polysorbate,

4. The pharmaceutical composition according to any one of claims 1 to 3, wherein the polysorbate is polysorbate 80 (PS80).

5. The pharmaceutical composition according to any one of claims 1 to 4, wherein the pharmaceutical composition comprises phytonadione in a concentration of about 2 mg/mL.

6. The pharmaceutical composition according to any one of claims 1 to 5, wherein the ratio $\frac{weight \left(non-ionic surfactant\right)}{weight \left(phytonadione\right)}$ is in the range of from about 6 to about 15, preferably the ratio $\frac{weight \left(non-ionic surfactant\right)}{weight \left(phytonadione\right)}$ is about 10 nm or about 13.

7. The pharmaceutical composition according to any one of claims 1 to 6, wherein the phase inversion temperature is in the range of from about 50 °C to about 80 °C.

8. The pharmaceutical composition according to any one of claims 1 to 7, wherein the pharmaceutical composition comprises a plurality of association colloids having a SPAN of in the range of from about 0.6 to about 0.9.

9. A method of manufacturing the pharmaceutical composition according to any one of claims 1 to 8, the method comprising the steps of:
a) mixing phytonadione and a non-ionic surfactant to provide a first mixture, and heating said first mixture to a first temperature T₁ to provide a first mixture having a temperature T₁, wherein T₁ is in the range of from about 50 °C to about 80 C;
b) dissolving an emulsifier in water for injection to provide a second mixture, and heating said second mixture to a second temperature T₂ to provide a second mixture having a temperature T₂, wherein T₂ is in the range of from about 50 °C to about 80 °C;
c) mixing said first mixture having a temperature T₁ obtained from step a) and said second mixture having a temperature T₂ obtained from step b) to provide a third mixture; and
d) cooling down said third mixture to a temperature T₃ in the range of from about 20 °C to about 30 °C to provide the pharmaceutical composition.

10. The method according to claim 9, wherein step c) is performed at a temperature T₄, wherein T₄ is in the range between T₁ and T₂.

11. The method according to claim 9 or 10, wherein the difference between T₁ and T₂ is about 10 °C or less.

12. The method according to any one of claims 9 to 11, wherein step d) further comprises a step of sterilizing the pharmaceutical composition.

13. The method according to any one of claims 9 to 12, wherein the first mixture contains less than about 3 wt.-% of water based on a total weight of the first mixture.

14. A container comprising the pharmaceutical composition according to any one of claims 1 to 8.

15. The container according to claim 1, wherein the container is a syringe, preferably a glass syringe.
